# EUROPEAN PATENT SPECIFICATION

(11) **EP 1 778 315 B1**
(45) Date of publication and mention of the grant of the patent: **11.03.2009**
(21) Application number: 05744991.0
(22) Date of filing: 17.05.2005
(51) Int. Cl.: A61M 3/02, A61M 31/00, A61F 11/00

(54) **DEVICE FOR SUPPLYING FLUID TO AN EAR**
VORRICHTUNG ZUR ZUFUHR VON FLÜSSIGKEIT ZU EINEM OHR
DISPOSITIF D"INJECTION DE FLUIDE DANS UNE OREILLE

(30) Priority: 15.07.2004 GB 0415808
(43) Date of publication of application: 02.05.2007
(73) Proprietor: Hill, Michael James, Hale WA15 9AX (GB); Llewellyn, Mark, Little Leigh, Northwich CW8 4RP (GB)
(72) Inventor: Hill, Michael James, Hale WA15 9AX (GB); Llewellyn, Mark, Little Leigh, Northwich CW8 4RP (GB)
(74) Representative: Bartle, Robin Jonathan
(86) International application number: PCT/GB2005/001904
(87) International publication number: WO 2006/008432

(56) References cited:
- WO-A-20/05055921
- BE-A- 365 554
- DE-A1- 10 323 565
- DE-U1- 9 112 982
- FR-A- 2 726 476
- US-A- 3 913 842
- US-A- 5 022 414
- US-A- 5 147 293
- US-A- 5 833 675
- US-A1- 2003 229 322
- US-A1- 2004 126 436
- US-B1- 6 432 117

## Description

The present invention relates to a device for dispensing a pre-defined quantity of fluid to an ear which is both simple in construction and easy to use.

A number of medical conditions are associated with that of the ear and whilst most conditions can be easily treated, frequently the treatment requires a fluid to be placed in the ear and this can be problematic, not least because when a person is upright, the fluid may drain from the ear. Commonly, the ears are treated by means of a medicament in the form of a fluid which can be dispensed to the ear with a device which dispenses the fluid in a drop wise manner. A device which is commonly used comprises a vial with a screw lid containing the medicament and the lid being adapted such that it also incorporates a pipette with a rubber nipple, which can then be used to dispense a quantity of the medicament into the ear. Usually a procedure using the device requires another person to accurately drop the medicament into the ear whilst a patient is lying on their side. Therefore, treatment of both ears can be problematic, as when the patient turns their head so that their other ear is presented in an upright position, the medicament may flow from the ear that was originally treated. A tissue or bung is usually required to maintain the fluid in the ear such that the medicament may be retained for an effective amount of time. Should a patient treat themselves, spillages can also result as it can be quit difficult to treat an area which is not visible. Furthermore, due to the utilitarian design of the pipettes, it can be most difficult to control the quantity of medicament being dispensed and this can be most problematic should a certain dose of medicament be required. Cleaning ears with ear buds can also cause problems with wax build up as the buds can compact rather than remove the wax and it is therefore preferable to use a method of wax removal that also alleviates this problem.

A number of devices have been proposed in order to address one or more of the problems associated with the commonly used devices. For example, US 4,995,867 discloses a aural medication administration device which allows a patient to self-administer aural medication to the ear disclosed in any plane. US 6,645,173 further discloses a medication dispensing device which can be attached to the head of an individual by a head band having a medicinal depositary reservoir located at the top of the head band, wherein a measured amount of liquid medication would be inserted therein and the device would hold the medication until released and dispensed into the ear canal through an ear piece. However, both devices are cumbersome in their use and require the medicaments to be inserted into each device prior to dispensing the medicament.

Prior art document BE365554 describes a device for carrying out the politzerization procedure. This involves providing compressed gas through one of the nostrils of a patient. The device in question is connectable to a compressed air or compressed oxygen container and has a nozzle insertable through the patient's nostril.

United States patent 5022414 discloses a tissue separator which way be used as an external ear cleaning device for wax removal by a fluid medium under pressure.

It is therefore an object of the present invention to alleviate one or more of the associated problems with prior art devices and to provide for a device which is capable of dispensing an accurate and pre-defined quantity of fluid to an ear which is simple in its construction and effectively expels a known quantity of fluid into the ear in a procedure which can be easily performed by a patient on their own.

In accordance with the present invention, there is provided a combination of an oil and an oil dispensing device for dispensing a pre-defined quantity of the oil to a user's auditory canal, the device comprising a vessel containing the oil, a nozzle adapted to be placed in the auditory canal, and a pump action mechanism operable by the user to draw the predefined quantity of oil from the vessel and expel it via the nozzle when the vessel and/or device is in a substantially upright or substantially inverted position, thereby atomizing the oil, wherein the oil comprises a mixture of a vegetable or nut oil with a mineral oil.

The device may permit the correct unit dose of an oil to be dispensed to the ear and it will be apparent to one skilled in the art that this will be important with regard to medicaments that require a given quantity of therapeutic agent for it's effective action.

It is preferred that the oil comprises a fixed oil, which includes fatty oils. Such oils commonly occur in nature and are derived from plants and comprise esters of glycerin and fatty acids. Vegetable or nut oils are used in accordance with the present invention, however, constituent(s) and/or derivative(s) of these oils may also be used. It should be understood that vegetable or nut oil is any oil or derivative that can be obtained from plants.

As the nozzle is adapted to be received in the ear (auditory canal), the device can be accommodated in the ear with comfort and it also ensures that the required therapeutic quantity of oil is placed in the desired part of the ear (such as just before the ear drum within the auditory canal). Preferably, the nozzle is disposed at an angle perpendicular to the vessel. under pressure. The device may further comprise a releasable pressurized valve.

The atomisation of the known volume of oil assists the action of the oil in ear.

It will be apparent that the device may be used for a number of uses and most common medical conditions of the ear stem from excess ear wax production which may be treated by a wax softening or dissolving agent. The device could also be utilized to supply oil in order to reduce or eradicate ingress of water (of any type) in the ear. The oil may be used to lubricate the ear, so as to facilitate the insertion of a medical device. The oil could also be deployed so to alleviate any discomfort associated with external dynamic or static ambient pressure changes, for example on aeroplane flights etc.

The use of a mineral oil may be to act as a thinner for a vegetable/nut oil. Preferably, a mineral oil is one that can be distilled down into a white mineral or petroleum oil or paraffin oil. More preferably, the mineral oil is Paraffinum Liquidum. Should a vegetable oil be used to soften or dissolve the ear wax, it will preferably be olive oil and this may be an oil approved by the relevant national or international agencies for use in the human and/or animal bodies. The oil may also comprise or additionally comprise oils derived from a range of organisms, such as animals and fish. A suitable vegetable oil, may comprise Tea Tree oil. The oil may also contain a pharmaceutically active ingredient and the oil may additionally comprise a pharmaceutically acceptable carrier, or the oil may act as such a carrier. An additive may also be provided with the oil to prevent oxidation and/or microbial contamination and such an additive may be a preservative, an anti-oxidant or an anti-microbial agent for example.

The nozzle may further comprise a rim that it goes around part of the nozzle so as to prevent the oil from leaving the ear during and/or after expulsion from the vessel. The nozzle may be manufactured from a number of materials but would be preferably manufactured from a plastic or rubber material. The device may be sealed so as to prevent tampering and an anti-tampering seal may also be provided so as to indicate whether or not tampering has occurred with the device. Preferably, the device dispenses a metered unit dose of the oil which relates to the quantity required for the successful treatment of an ear condition.

In accordance with a further aspect of the present invention, there is provided a kit of parts for the production of an oil dispensing device for dispensing a pre-defined quantity of oil to an ear, the kit comprising;
(a) a vessel for holding oil;
(b) a nozzle for insertion in a user's auditory canal, adapted to be coupled to the vessel and capable of atomizing and expelling the oil;
(c) a pump action mechanism for actuating the expulsion of a pre-defined quantity of oil from the vessel via the nozzle when the vessel and/or device is in a substantially upright or substantially inverted position, thereby atomizing the oil; and
(d) an oil comprising a mixture of a vegetable/nut and a mineral oil.

The present invention will now be more fully described by way of example only with reference to the following figure.

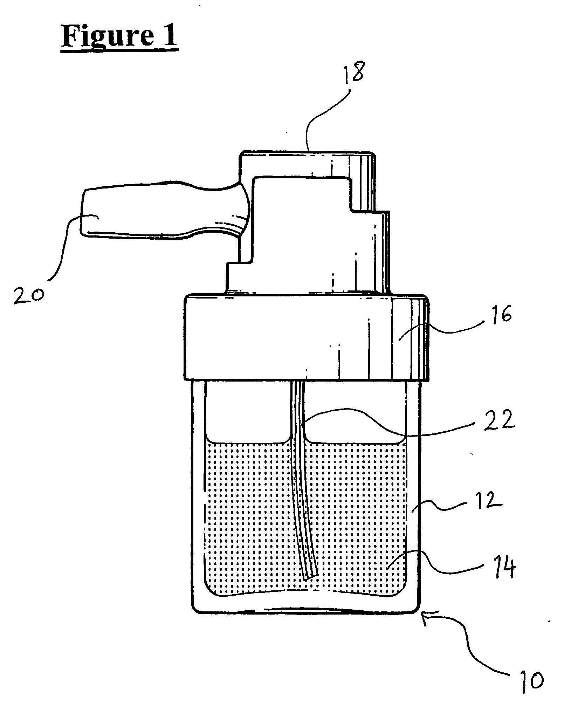

Figure 1 shows a device in accordance with the present invention.

With reference to Figure 1, there is provided a device 10 for dispensing an ear wax softening agent to the ear, the device having a vessel 12 containing olive oil 14. The vessel 12 has a cap 16 that seals the vessel and incorporates a pump-action mechanism for expelling the oil 14 and the mechanism is actuated by button 18 which in turn is attached to nozzle 20. An aperture (not shown) runs through the nozzle 20 and permits the oil 14 from being expelled from the device 10. The pump-action mechanism (not shown) includes a capillary tube 22 through which the oil 14 can be expelled from the vessel 12 through the nozzle 20. In the case of a device manufactured for actuation whereby the vessel is inverted, the capillary tube 22 is not required.

In use, the vessel 10 will be manufactured as a sealed device containing a quantity of olive oil 14 for dispensing to the ear so as to soften and/or dissolve the ear wax prior to or in addition to syringing the ear with water or a saline solution. The device 10 can be used in an upright position and by the patient themselves to dispense the oil as required. The pump action mechanism draws the oil 14 up the capillary tube 22 when the button 18 is depressed which expels a desired quantity of oil to the ear via the nozzle 20 which is adapted to fit snugly within the ear. The nozzle 20 may also have a disc disposed around its circumference so as to ensure that the oil 14 does not exit the ear during its application. The device can be made out of a number of materials, but commonly the vessel 12 will be produced from a plastics or glass material with the lid 16 being sealed onto it. The device 10 may also have a label disposed thereon to provide patient information such as dosing etc.

The device 10 can also be adapted for dispensing pharmaceutical agents.

## Claims

1. A combination of an oil (14) and an oil dispensing device (10) for dispensing a pre-defined quantity of the oil to a user's auditory canal, the device comprising a vessel (12) containing the oil, a nozzle (20) adapted to be placed in the auditory canal, and a pump action mechanism operable by the user to draw the predefined quantity of oil from the vessel and expel it via the nozzle when the vessel and/or device is in a substantially upright or substantially inverted position, thereby atomizing the oil, wherein the oil comprises a mixture of a vegetable or nut oil with a mineral oil.

2. A device as claimed in the preceding claim, wherein the pump action mechanism is actuable by means of a button (18).

3. A combination as claimed in claim 1 or claim 2, wherein the nozzle is disposed at an angle perpendicular to the longitudinal axis of the vessel.

4. A combination as claimed in any preceding claim, wherein the nozzle further comprises a rim that extends around the nozzle so as to prevent the oil from leaving the ear during and/or after expulsion from the vessel.

5. A combination as claimed in any preceding claim, wherein the nozzle is manufactured from a plastics or rubber material.

6. A combination as claimed in any preceding claim, wherein the device is sealed.

7. A combination as claimed in any preceding claim, wherein the device further comprises a metering valve, through which the pre-defined quantity of oil is dispensed.

8. A combination as claimed in any preceding claim, wherein the vegetable oil comprises two or more vegetable oils.

9. A combination as claimed in any preceding claim, wherein the mineral oil comprises a white mineral oil.

10. A combination as claimed in any preceding claim, wherein the mineral oil comprises two or more mineral oils.

11. A combination as claimed in any preceding claim, wherein the vegetable oil comprises olive oil.

12. A combination as claimed in any one of claims 1 to 9, wherein the vegetable oil comprises Tea Tree oil.

13. A combination as claimed in any preceding claim, wherein the oil further comprises a pharmaceutically active ingredient and/or a pharmaceutically acceptable carrier.

14. A combination as claimed in any preceding claim, wherein the oil further comprises an additive to prevent oxidation and/or microbial contamination.

15. A combination as claimed in any preceding claim for use in softening or dissolving ear wax.

16. A combination as claimed in any one of claims 1 to 14 for use in reducing or eradicating ingress of water in the ear.

17. A combination as claimed in any one of claims 1 to 14 for use in lubricating any part of the ear.

18. A combination as claimed in any one of claims 1 to 14 for use in lubricating the ear, so as to facilitate the insertion of a medical device.

19. A combination as claimed in any one of claims 1 to 14 for use in alleviating discomfort associated with external dynamic or static ambient pressure changes.

20. A kit of parts for the production of an oil dispensing device (10) for dispensing a pre-defined quantity of oil to an ear, the kit comprising;
(a) a vessel (12) for holding oil (14);
(b) a nozzle (20) for insertion in a user's auditory canal, adapted to be coupled to the vessel and capable of atomizing and expelling the oil;
(c) a pump action mechanism for actuating the expulsion of a pre-defined quantity of oil from the vessel via the nozzle when the vessel and/or device is in a substantially upright or substantially inverted position, thereby atomizing the oil; and
(d) an oil (14) comprising a mixture of a vegetable/nut and a mineral oil.

## Patentansprüche

1. Kombination aus einem Öl (14) und einer Ölausgabevorrichtung (10) zum Ausgeben einer vorbestimmten Menge des Öls in den Gehörgang eines Benutzers, wobei die Vorrichtung ein das Öl enthaltendes Gefäß (12), eine Düse (20), die zum Platzieren im Gehörgang eingerichtet ist, und einen Pumptätigkeit bereitstellenden Mechanismus aufweist, der durch den Benutzer zum Ziehen der vorbestimmten Menge von Öl aus dem Gefäß und Ausstoßen desselben über die Düse bedienbar ist, wenn das Gefäß und/oder die Vorrichtung sich in einer im Wesentlichen aufrechten oder im Wesentlichen umgedrehten Position befindet, wodurch das Öl zerstäubt wird, wobei das Öl eine Mischung aus einem Pflanzen- oder Nussöl mit einem Mineralöl aufweist.

2. Vorrichtung nach dem vorhergehenden Anspruch, bei der der Pumptätigkeit bereitstellende Mechanismus mittels eines Schalters (18) betätigt werden kann.

3. Kombination nach Anspruch 1 oder Anspruch 2, bei der die Düse in einem Winkel senkrecht zu der Längsachse des Gefäßes angeordnet ist.

4. Kombination nach einem vorhergehenden Anspruch, bei der die Düse weiter einen Rand aufweist, der sich um die Düse herum erstreckt, um zu verhindern, dass das Öl das Ohr während und/oder nach Ausstoßen aus dem Gefäß verlässt.

5. Kombination nach einem vorhergehenden Anspruch, bei der die Düse aus einem Kunststoff oder Gummimaterial hergestellt wird.

6. Kombination nach einem vorhergehenden Anspruch, bei der die Vorrichtung versiegelt ist.

7. Kombination nach einem vorhergehenden Anspruch, bei der die Vorrichtung weiter ein Dosierventil aufweist, durch das die vorbestimmte Menge von Öl ausgegeben wird.

8. Kombination nach einem vorhergehenden Anspruch, bei der das Pflanzenöl zwei oder mehr Pflanzenöle enthält.

9. Kombination nach einem vorhergehenden Anspruch, bei der das Mineralöl ein weißes Mineralöl aufweist.

10. Kombination nach einem vorhergehenden Anspruch, bei der das Mineralöl zwei oder mehr Mineralöle aufweist.

11. Kombination nach einem vorhergehenden Anspruch, bei der das Pflanzenöl Olivenöl aufweist.

12. Kombination nach einem der Ansprüche 1 bis 9, bei der das Gemüseöl Teebaumöl aufweist.

13. Kombination nach einem vorhergehenden Anspruch, bei der das Öl weiter einen phannazeutischen Wirkstoff und/oder einen pharmazeutisch akzeptablen Träger aufweist.

14. Kombination nach einem vorhergehenden Anspruch, bei der das Öl weiter einen Zusatzstoff aufweist, um Oxidation und/oder mikrobielle Kontamination zu verhindern.

15. Kombination nach einem vorhergehenden Anspruch, zum Gebrauch beim Erweichen oder Auflösen von Ohrenschmalz.

16. Kombination nach einem der Ansprüche 1 bis 14, zum Gebrauch bei Reduzieren oder Verhindern des Eindringens von Wasser in das Ohr.

17. Kombination nach einem der Ansprüche 1 bis 14, zum Gebrauch beim Schmieren irgendeines Teils des Ohrs.

18. Kombination nach einem der Ansprüche 1 bis 14, zum Gebrauch beim Schmieren des Ohrs, um so die Einführung einer medizinischen Einrichtung zu vereinfachen.

19. Kombination nach einem der Ansprüche 1 bis 14, zum Gebrauch beim Lindern von Beschwerden, die mit externen dynamischen oder statischen Umgebungsdruckänderungen verknüpft sind.

20. Teilesatz für die Herstellung einer Ölausgabevorrichtung (10) zum Ausgeben einer vorbestimmten Menge von Öl in ein Ohr, wobei der Satz Folgendes aufweist:
(a) ein Gefäß (12) zum Halten von Öl (14);
(b) eine Düse (20) zur Einführung in den Gehörgang eines Benutzers, die eingerichtet ist, um an das Gefäß gekoppelt zu werden und das Öl zerstäuben und ausstoßen kann;
(c) einen Pumptätigkeit bereitstellenden Mechanismus zum Bewirken des Ausstoßes einer vorbestimmten Menge von Öl aus dem Gefäß über die Düse, wenn das Gefäß und/oder die Einrichtung sich in einer im Wesentlichen aufrechten oder im Wesentlichen umgedrehten Position befindet, wodurch das Öl zustäubt wird; und
(d) ein Öl (14), das eine Mischung aus einem Pflanzen-/Nuss- und einem Mineralöl aufweist.

## Revendications

1. Combinaison constituée d'une huile (14) et d'un dispositif de distribution d'huile (10) permettant de distribuer une quantité prédéfinie de l'huile au niveau du canal auditif d'un utilisateur, le dispositif comportant un récipient (12) contenant l'huile, une buse (20) adaptée pour être placée dans le canal auditif, et un mécanisme d'action de pompe pouvant être actionné par l'utilisateur pour aspirer la quantité prédéfinie d'huile en provenance du récipient et l'expulser par le biais de la buse quand le récipient et / ou le dispositif est dans une large mesure à la verticale ou dans une large mesure en position inversée, pour de ce fait atomiser l'huile, dans laquelle l'huile comporte un mélange constitué d'une huile végétale ou d'arachide et d'une huile minérale.

2. Combinaison selon la revendication précédente, dans laquelle le mécanisme d'action de pompe est actionnable au moyen d'un bouton (18).

3. Combinaison selon la revendication 1 ou la revendication 2, dans laquelle la buse est disposée en un angle perpendiculaire à l'axe longitudinal du récipient.

4. Combinaison selon l'une quelconque des revendications précédentes, dans laquelle la buse comporte par ailleurs un bord qui s'étend autour de la buse de manière à empêcher l'huile de sortir de l'oreille au cours de l'expulsion en provenance du récipient ou après celle-ci.

5. Combinaison selon l'une quelconque des revendications précédentes, dans laquelle la buse est fabriquée à partir d'un matériau en plastique ou en caoutchouc.

6. Combinaison selon l'une quelconque des revendications précédentes, dans laquelle le dispositif est fermé hermétiquement.

7. Combinaison selon l'une quelconque des revendications précédentes, dans laquelle le dispositif comporte par ailleurs une valve doseuse, au travers de laquelle la quantité prédéfinie d'huile est distribuée.

8. Combinaison selon l'une quelconque des revendications précédentes, dans laquelle l'huile végétale comporte deux huiles végétales ou plus.

9. Combinaison selon l'une quelconque des revendications précédentes, dans laquelle l'huile minérale comporte une huile minérale blanche.

10. Combinaison selon l'une quelconque des revendications précédentes, dans laquelle l'huile minérale comporte deux huiles minérales ou plus.

11. Combinaison selon l'une quelconque des revendications précédentes, dans laquelle l'huile végétale comporte de l'huile d'olive.

12. Combinaison selon l'une quelconque des revendications 1 à 9, dans laquelle l'huile végétale comporte de l'huile essentielle de Melaleuca alternifolia.

13. Combinaison selon l'une quelconque des revendications précédentes, dans laquelle l'huile comporte par ailleurs un ingrédient pharmaceutiquement actif et / ou une substance inerte de qualité pharmaceutique.

14. Combinaison selon l'une quelconque des revendications précédentes, dans laquelle l'huile comporte par ailleurs un additif permettant d'empêcher l'oxydation et / ou la contamination microbienne.

15. Combinaison selon l'une quelconque des revendications précédentes servant à ramollir ou à dissoudre le cérumen.

16. Combinaison selon l'une quelconque des revendications 1 à 14 servant à réduire ou à éradiquer toute pénétration d'eau dans l'oreille.

17. Combinaison selon l'une quelconque des revendications 1 à 14 servant à lubrifier toute partie de l'oreille.

18. Combinaison selon l'une quelconque des revendications 1 à 14 servant à lubrifier l'oreille, dans le but de faciliter l'insertion d'un dispositif médical.

19. Combinaison selon l'une quelconque des revendications 1 à 14 servant à soulager toute sensation de gêne associée à tout changement de pression ambiante dynamique ou statique externe.

20. Kit de pièces permettant de produire un dispositif de distribution d'huile (10) permettant de distribuer une quantité prédéfinie d'huile au niveau d'une oreille, le kit comportant :
(a) un récipient (12) destiné à renfermer l'huile (14) ;
(b) une buse (20) à des fins d'insertion dans le canal auditif d'un utilisateur, adaptée pour être accouplée au récipient et capable d'atomiser et d'expulser l'huile ;
(c) un mécanisme d'action de pompe destiné à actionner l'expulsion d'une quantité prédéfinie d'huile en provenance du récipient par le biais de la buse quand le récipient et / ou le dispositif est dans une large mesure à la verticale ou dans une large mesure en position inversée, pour de ce fait atomiser l'huile ; et
(d) une huile (14) comportant un mélange constitué d'une huile végétale / d'arachide et d'une huile minérale.
